# EUROPÄISCHE PATENTSCHRIFT

(11) **EP 0 449 110 B1**
(45) Veröffentlichungstag und Bekanntmachung des Hinweises auf die Patenterteilung: **05.06.1996**
(21) Anmeldenummer: 91104409.7
(22) Anmeldetag: 21.03.1991
(51) Int. Cl.: C07C 265/14, C07C 271/20, C07C 271/04, A01N 47/12, C08G 18/00

(54) **Neue 2,2-Dialkylpentan-1,5-diisocyanate, -diurethane und -dicarbamidsäure-chloride, Verfahren zu ihrer Herstellung und ihre Verwendung**
2,2-Dialkylpentane-1,5-diisocyanates, -diurethanes, and -dicarbamic acid chlorides, process for their preparation and their use
2,2-Dialkylpentane-1,5-diisocyanates, -diuréthanes, et chlorures d'acides dicarbamiques, leur procédé de préparation et leur usage

(30) Priorität: 30.03.1990 DE 4010226
(43) Veröffentlichungstag der Anmeldung: 02.10.1991
(73) Patentinhaber: BASF Aktiengesellschaft, D-67063 Ludwigshafen (DE)
(72) Erfinder: Merger, Franz, Dr., W-6710 Frankenthal (DE); Otterbach, Andreas, Dr., W-6710 Frankenthal (DE); Witzel, Tom, Dr., W-6700 Ludwigshafen (DE); Renz, Hans, Dr., W-6701 Meckenheim (DE)

(56) Entgegenhaltungen:
- EP-A- 0 077 105
- EP-A- 0 261 604
- DE-A- 2 830 243
- US-A- 3 631 198
- US-A- 3 691 134
- JUSTUS LIEBIGS, ANNALEN DER CHEMIE, Band 562, 1949, Seiten 75-136; W. SIEFKEN: "Mono- und polyisocyanate"

## Beschreibung

Die Erfindung betrifft neue 2,2-Dialkylpentan-1,5-diisocyanate, Verfahren zu ihrer Herstellung und Verwendung, vorzugsweise zur Herstellung von kompakten oder zelligen Kunststoffen nach dem Polyisocyanat-polyadditionsverfahren sowie 2,2-Dialkylpentan-1,5-diurethane und 2,2-Dialkylpentan-1,5-dicarbamidsäurechloride, die sich z.B. als Ausgangsstoffe zur Herstellung der 2,2-Dialkylpentan-1,5-diisocyanate oder von höhermolekularen Polykondensationsprodukten eignen und Schädlingsbekämpfungsmittel sind. Im Verlaufe der Beschreibung ist zur Vereinfachung üblicherweise nur von 2,2-"Dialkyl"pentangruppen die Rede, obgleich unter dieser Bezeichnung im Sinne der Erfindung gleichermaßen die 2,2-Dialkenylpentan- und 2-Alkyl-2-alkenylpentangruppen und 1-Methylen-1-propylen-cycloalkylenreste mit 5 bis 8 C-Atomen zu verstehen sind.

Organische, beispielsweise aliphatische, cycloaliphatische oder aromatische Polyisocyanate sind aus zahlreichen Patent- und Literaturveröffentlichungen, wie z.B. von Werner Siefken, Ann. 562, 75-136 (1949), bekannt und finden vorzugsweise Verwendung als hochreaktive Zwischenprodukte und Ausgangsstoffe zur Herstellung von Urethan-, Harnstoff- und/oder Isocyanuratgruppen enthaltenden hochmolekularen Polyadditionsprodukten.

Beispielhaft beschrieben werden u.a. Pentamethylen- und 2,2-Dimethyl-pentamethylen-diisocyanat, die durch Phosgenierung der entsprechenden Diamine und thermische Spaltung der intermediär gebildeten Dicarbamidsäurechloride hergestellt werden können. Von großtechnischer Bedeutung sind insbesondere die (cyclo)aliphatischen Diisocyanate 1,6-Hexamethylen-diisocyanat und 1-Isocyanato-3-isocyanatomethyl-(3,5,5-trimethyl)-cyclohexan (Isophoron-diisocyanat) als Aufbaukomponenten für Fasern, Klebstoffe oder licht- und thermostabile Überzüge und Lacke und die aromatischen Polyisocyanate 1,5-Naphthylen-diisocyanat als Ausgangsstoffe für kompakte oder zellige Elastomere und Gießharze, 2,4- und 2,6-Toluylen-diisocyanat und Mischungen davon und/oder Mischungen aus Diphenylmethan-diisocyanaten und Polyphenyl-polymethylen-polyisocyanaten als Ausgangsstoffe für weichelastische, halbharte oder harte Polyurethanschaumstoffe oder Isocyanurat- und Urethangruppen enthaltende Hartschaumstoffe und 4,4'-Diphenylmethan-diisocyanat als Ausgangsstoff für Fasern, Klebstoffe und thermoplastische Polyurethane.

Außerdem sind noch eine Vielzahl von Patentschriften bekannt, in denen spezielle Herstellungsverfahren für organische Polyisocyanate oder spezielle organische Polyisocyanate für spezielle Anwendungsgebiete beschrieben werden.

Nach Angaben der US-A-3 311 654 werden z.B. organische Isocyanate, beispielsweise Monoisocyanate, Diisocyanate und höherfunktionelle Polyisocyanate durch thermische Spaltung von sekundären Carbamidsäurechloriden hergestellt. Die DE-C-28 30 243 beschreibt aliphatische, verzweigte langkettige Diisocyanate, nämlich 1,9-Diisocyanato-5-methyl-nonan und 1,8-Diisocyanato-2,4-dimethyloctan, als geeignete Diisocyanate für Coil-Coating-Lacke. Mehrfach substituierte 1,5-Pentandiisocyanate mit einer sterisch behinderten Isocyanatgruppe der Formel

OCN-CHR¹-CR²R³-CH₂-CHR⁴-CH₂-NCO ,

in der u.a. R¹ eine C₁- bis C₁₀-Alkylgruppe, eine Phenylgruppe oder eine Alkoxyalkylengruppe ist, sind Gegenstand der EP-B-077 105 und finden Verwendung in Lacksystemen mit einer verlängerten Verarbeitungszeit.

Die US-A-3 691 134 nennt als geeignetes Cycloalkyldiisocyanat zur Herstellung von Polyurethanen u.a. das 1-(Isocyanatomethyl)-1-(3-isocyanatopropyl)-cyclohexan.

Isocyanato-uretdione, die hergestellt werden durch Dimerisierung von 2-Methylpentan-1,5-diisocyanat oder 2-Ethylbutan-1,4-diisocyanat und in der Hitze zu mehr als 70 % rückspaltbar sind, sind bekannt aus der DE-A-32 27 779 (US-A-4 668 780). Die modifizierten Diisocyanate finden insbesondere Verwendung in lösungsmittelhaltigen oder -freien Ein- und Zweikomponenten-Lacken, wie z.B. Coil-Coating- und High Solid-Lacken und Polyurethan-Pulverlacken. Das als Ausgangsstoff geeignete 2-Methyl-pentan-1,5-diisocyanat wird z.B. nach Angaben der US-A-3 631 198 aus 1,5-Diamino-2-methylpentan durch Phosgenierung in niedermolekularen Phthalsäuredialkylester als Lösungsmittel hergestellt. Beschrieben in der EP-A-261 604 (US-A-4 748 226) werden ferner 2-Alkoxymethylpentan-1,5-diisocyanate, die durch geeignete Modifizierung der Alkoxymethylgruppe dem Verwendungszweck und den Verarbeitungsbedingungen angepaßt werden können. In den genannten Publikationen nicht beschrieben werden 2,2-Dialkylpentan-1,5-diisocyanate, -1,5-diurethane und -1,5-dicarbamidsäurechloride und ein geeignetes Verfahren zu ihrer Herstellung.

Der Erfindung liegt die Aufgabe zugrunde, neue Pentan-diisocyanate zur Verfügung zu stellen, die durch geeignete Wahl der Substituenten hinsichtlich ihrer physikalischen Eigenschaften und ihrer Verarbeitbarkeit modifizierbar sind, die die mechanischen Eigenschaften daraus hergestellter Polyisocyanat-polyadditionsprodukte beeinflussen können und die als 2,2-Dialkylpentan-1,5-diurethane oder -1,5-dicarbamidsäurechloride als Schädlingsbekämpfungsmittel wirksam sein könnten. Die Produkte sollten auf einfache Weise kostengünstig hergestellt werden können.

Diese Aufgabe wird gelöst mit 2,2-Dialkylpentan-1,5-diisocyanaten der Formel (I) 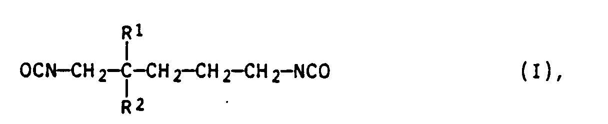
in der R¹ und R² gleich oder verschieden sind und bedeuten:
einen linearen C₁- bis C₁₂-Alkylrest, vorzugsweise einen C₁- bis C₉-Alkylrest,
einen verzweigten C₃- bis C₁₂-Alkylrest, vorzugsweise einen C₃- bis C₈-Alkylrest,
einen linearen C₂- bis C₁₂-Alkenylrest, vorzugsweise einen C₂- bis C₉-Alkenylrest oder
einen verzweigten C₄- bis C₁₂-Alkenylrest, vorzugsweise einen C₄- bis C₈-Alkenylrest, wobei das 2,2-Dimethylpentan-1,5-diisocyanat ausgenommen ist.

Besonders bewährt haben sich und daher vorzugsweise Anwendung finden Diisocyanate der Formel (I), in der R¹ und R² gleich oder vorzugsweise verschieden und ausgewählt sind unter Alkylresten, wie z.B. dem Methyl-, Ethyl-, n-Propyl-, Isopropyl-, n-Butyl-, Isobutyl-, sec.-Butyl-, 2-Methylbutyl-, n-Pentyl-, Neopentyl-, 2-Methylpentyl-, n-Hexyl-, 2-Ethylhexyl-, n-Octyl-, n-Decyl- oder n-Dodecylrest, Alkenylresten, wie z.B. dem Propenyl-, 1- oder 2-Butenyl-, 1-Hexenyl-, 1-Octenyl oder 2-Vinyl-octylrest und Alkylenresten wie z.B. dem Butylen-, Pentylen- oder Hexylenrest.

Als bevorzugte 2,2-Dialkylpentan-1,5-diisocyanate seien beispielsweise genannt: 2,2-Diethyl-, 2,2-Dipropyl-, 2,2-Dibutyl-, 2,2-Dihexyl-, 2,2-Dioctyl-, 2,2-Diisopropyl-pentan-1,5-diisocyanat, 2-Methyl-2-ethyl-, 2-Methyl-2-n-propyl-, 2-Methyl-2-isopropyl-, 2-Ethyl-2-propyl-, 2-Ethyl-2-butyl-, 2-Ethyl-2-hexyl-, 2-Ethyl-2-octyl- und 2-Ethyl-2-decyl-pentan-1,5-diisocyanat, 2-Methyl-2-propenyl- und 2-Ethyl-2-butenylpentan-1,5-diisocyanat. Insbesondere Anwendung finden 2-Methyl-2-propylpentan-, 2-Ethyl-2-butylpentan- und 2- Methyl-2-n-nonyl-pentan-1,5-diisocyanat.

Die 2,2-Dialkylpentan-1,5-diisocyanate der Formel (I) sind erhältlich durch thermische Spaltung von 2,2-Dialkylpentan-1,5-dicarbamidsäurechloriden der Formel (III) 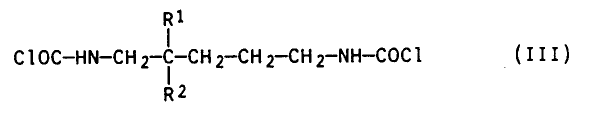
in der R¹ und R² die vorgenannte Bedeutung besitzen, in Gegenwart von unter den Reaktionsbedingungen gegenüber NCO-Gruppen inerten organischen Lösungsmitteln bei einer Temperatur im Bereich von 80 bis 200°C, vorzugsweise 120 bis 200°C in die erfindungsgemäßen 2,2-Dialkylpentan-1,5-diisocyanate und Chlorwasserstoff.

Vorzugsweise werden die 2,2-Dialkylpentan-1,5-diisocyanate der Formel (I) jedoch hergestellt durch thermische Spaltung von 2,2-Dialkylpentan-1,5-diurethanen der Formel (II) 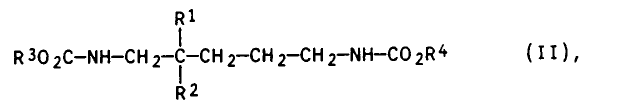
in der R¹ und R² die obengenannte Bedeutung besitzen und R³ und R⁴ gleich oder verschieden sind und bedeuten
einen linearen C₁- bis C₂₀-Alkylrest, vorzugsweise einen linearen C₁- bis C₁₀-Alkylrest und insbesondere einen linearen C₃- bis C₆-Alkylrest, einen verzweigten C₃- bis C₂₀-Alkylrest, vorzugsweise einen verzweigten C₃- bis C₁₀-Alkylrest und insbesondere einen verzweigten C₃- bis C₆-Alkylrest oder
einen C₅- bis C₁₂-Cycloalkylrest und vorzugsweise einen C₅- bis C₈-Cycloalkylrest,
in Gegenwart oder Abwesenheit von Katalysatoren
a) in der Gasphase bei Temperaturen von über 300°C unter vermindertem Druck oder
b) in flüssiger Phase bei Temperaturen von 175 bis 350°C
in die erfindungsgemäßen 2,2-Dialkylpentan-1,5-diisocyanate und Alkohole.

Die 2,2-Dialkylpentan-1,5-diurethane der Formel (II) und -dicarbamidchloride der Formel (III) sind erhältlich durch Umsetzung von 2,2-Dialkylpentan-1,5-diaminen der Formel (IV) 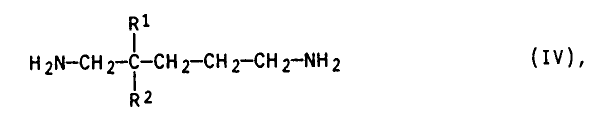
in der R¹ und R² die vorgenannte Bedeutung besitzen, in Gegenwart oder Abwesenheit von Katalysatoren mit
a) Harnstoff und einem primären und/oder sekundären Alkohol R³OH bzw. R⁴OH oder
b) Harnstoff und einem primären und/oder sekundären Alkohol R³OH bzw. R⁴OH, wobei die Reste R³ und R⁴ die obengenannte Bedeutung haben, in Gegenwart von Carbamidsäurealkylestern und/oder Dialkylcarbonaten
und gegebenenfalls Abtrennung des gebildeten Ammoniaks bzw. durch Phosgenierung der 2,2-Dialkylpentan-1,5-diamine der Formel (IV) oder deren Salze, vorzugsweise der 2,2-Dialkylpentan-1,5-diaminhydrochloride, in Lösungs- oder Verdünnungsmitteln.

Die neuen erfindungsgemäßen 2,2-Dialkylpentan-1,5-diisocyanate sowie die Ausgangsstoffe 2,2-Dialkylpentan-1,5-diurethane und -dicarbamidsäurechloride zu ihrer Herstellung und die Vorprodukte hierfür können beispielsweise nach folgenden Verfahren hergestellt werden:

2,2-Dialkylpentan-1,5-diamine der Formel (IV) sind z.B. dadurch erhältlich, daß man 2,2-dialkylsubstituierte 4-Cyanobutanale der Formel (V) 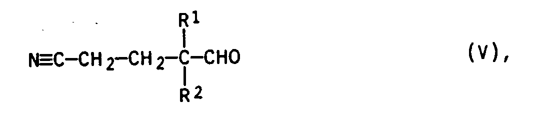
in der R¹ und R² die obengenannte Bedeutung besitzen, in einer ersten Reaktionsstufe mit überschüssigem Ammoniak an aciden Heterogenkatalysatoren aminiert und danach
die entstandenen Reaktionsprodukte in einer zweiten Reaktionsstufe mit Wasserstoff in Gegenwart von überschüssigem Ammoniak zu cobalt-, nickel-, ruthenium- und/oder anderen edelmetallhaltigen Katalysatoren, gegebenenfalls mit basischen Komponenten oder auf basischen oder neutralen Trägern hydriert.

Nach dem zweckmäßigerweise zur Anwendung kommenden Verfahren werden die Umsetzung mit Ammoniak und die nachfolgende Hydrierung in zwei räumlich voneinander getrennten Reaktionsräumen durchgeführt.

In einer ersten Verfahrensstufe setzt man die 2,2-disubstituierten 4-Cyanobutanale (V) mit überschüssigem Ammoniak um. Dabei hält man einen Druck von 15 bis 500 bar, vorzugsweise von 100 bis 350 bar und eine Temperatur von 20 bis 150°C, vorzugsweise von 30 bis 100°C ein. Die Kondensation wird in Gegenwart von aciden Heterogenkatalysatoren durchgeführt. Als acide Heterogenkatalysatoren eignen sich Metallverbindungen mit Lewissäuren-oder Brönstedtsäure-Charakter wie z.B. Aluminiumoxid, Siliciumdioxid, Titandioxid, Zirkondioxid, ferner Phosphate, wie z.B. Aluminiumphosphate oder Silikate wie z.B. amorphe oder kristalline Aluminosilikate. Bevorzugt verwendet man Aluminiumoxid, Titandioxid, Zirkondioxid und Siliciumdioxid, insbesondere Aluminiumoxid und Titandioxid. Die Acidität der Katalysatoren kann gegebenenfalls durch Dotierung mit Halogeniden erhöht werden. So finden z.B. auch halogendotierte Katalysatoren, wie Chlorid auf Aluminiumoxid oder Chlorid auf Titandioxid, Verwendung.

Bei der Umsetzung der 2,2-disubstituierten 4-Cyanobutanale (V) an den aciden Heterogenkatalysatoren hält man eine Katalysatorbelastung von 0,01 bis 10, vorzugsweise 0,02 bis 5, besonders bevorzugt von 0,05 bis 3 kg 2,2-disubstituiertem 4-Cyanobutanal pro kg Katalysator und Stunde ein. Pro Mol 2,2-disubstituiertem 4-Cyanobutanal setzt man zweckmäßig, aber nicht zwingend 5 bis 500 Mol NH₃, bevorzugt 30 bis 400 Mol, besonders bevorzugt 50 bis 300 Mol ein. Die Umsetzung der 4-Cyanobutanale mit Ammoniak kann auch in der Anwesenheit von inerten Lösungsmitteln, wie Alkanolen oder Tetrahydrofuran erfolgen.

Die Umsetzung der 2,2-disubstituierten 4-Cyanobutanale (V) läßt sich diskontinuierlich, bevorzugt kontinuierlich durchführen, z.B. in Druckbehältern oder Druckbehälterkaskaden. Nach einer besonders bevorzugten Ausführungsform werden die 2,2-disubstituierten 4-Cyanobutanale und NH₃ durch einen Rohrreaktor geleitet, in dem der Katalysator in Form eines festen Bettes angeordnet ist.

Die Gesamtverweilzeit in Verfahrensstufe 1 ergibt sich aus der Katalysatorbelastung und der Menge an eingesetztem Ammoniak. Sie liegt zweckmäßig im Bereich von 0,5 bis 120 Minuten, bevorzugt von 1 bis 40, besonders bevorzugt von 1,5 bis 20 Minuten.

Das so erhaltene Produkt wird in einer zweiten Verfahrensstufe mit 3 bis 10.000 Moläquivalenten Wasserstoff, bevorzugt 4,5 bis 30, gegebenenfalls nach Zufuhr von weiterem Ammoniak, einer katalytischen Hydrierung zugeführt.

Die Hydrierung wird vorzugsweise in flüssigem Ammoniak durchgeführt. Pro Mol in Verfahrensstufe 1 eingesetztem 2,2-disubstituierten 4-Cyanobutanal (V) verwendet man 5 bis 500 Mol NH₃, bevorzugt 30 bis 400 Mol, besonders bevorzugt 50 bis 300 Mol. Der NH₃-Anteil kann gegebenenfalls durch Zufuhr von NH₃ auf den gewünschten Wert erhöht werden.

Man hydriert im allgemeinen bei einer Temperatur von 60 bis 150°C, bevorzugt von 70 bis 140°C, besonders bevorzugt von 80 bis 130°C und einem Druck von 50 bis 500 bar, bevorzugt von 100 bis 350 bar, besonders bevorzugt von 150 bis 300 bar.

Die Katalysatorbelastungen liegen zweckmäßig im Bereich von 0,01 bis 5 kg/[kg·h], bevorzugt bei 0,02 bis 2,5 kg/[kg·h], besonders bevorzugt bei 0,05 bis 2 kg/[kg·h].

Bei kontinuierlicher Hydrierung ohne Produktrückführung ergibt sich die Gesamtverweilzeit aus der Katalysatorbelastung und der Menge an eingesetztem Ammoniak. Sie liegt im Bereich von 0,5 bis 120 Minuten, bevorzugt von 1 bis 40 Minuten, besonders bevorzugt bei 1,5 bis 20 Minuten.

Bei der Hydrierung können prinzipiell alle gängigen Hydrierkatalysatoren eingesetzt werden, die Nickel, Cobalt, Eisen, Kupfer, Ruthenium oder andere Edelmetalle der VIII. Nebengruppe des Periodensystems enthalten. Bevorzugt verwendet man Ruthenium-, Cobalt- oder Nickel-Katalysatoren. Besonders bevorzugt sind Ruthenium- und Cobalt-Katalysatoren. Die katalytisch aktiven Metalle können als Vollkontakte oder auf Trägern eingesetzt werden. Als solche Träger kommen z.B. Aluminiumoxid, Titandioxid, Zirkondioxid, Zinkoxid oder Magnesiumoxid/ Aluminiumoxide in Frage, bevorzugt werden Hydrierkatalysatoren mit basischen Komponenten, wie Oxide und Hydroxide von Alkali- und Erdalkalimetallen. Besonders bevorzugt sind daher basische Träger, wie z.B. β-Aluminiumoxid oder Mangesiumoxid/Aluminiumoxide. Besonders bevorzugt ist Magnesiumoxid/Aluminiumoxid mit einem Magnesiumoxidanteil von 5 bis 40 Gew.-%. Dabei kann der Magnesiumoxid und Aluminiumoxide enthaltende Träger amorph sein oder als Spinell vorliegen.

Besonders bevorzugt verwendet man in der Hydrierung Cobalt oder Ruthenium mit basischer Komponente oder Ruthenium. Diese Katalysatoren erhält man technisch in an sich bekannter Weise. So gewinnt man z.B. Ruthenium auf basischem Träger durch Auftragen von wäßrigen Rutheniumsalz-Lösungen wie Rutheniumchlorid und Rutheniumnitrat auf den entsprechenden Träger. Die Ruthenium-Konzentration auf dem Träger beträgt 0,1 bis 10 Gew.-% bevorzugt 0,5 bis 5 Gew.-%, besonders bevorzugt 1 bis 4 Gew.-%.

Nach Trocknung und gegebenenfalls nach Calcinierung bei Temperaturen von 120 bis 500°C, bevorzugt bei 200 bis 400°C, erfolgt die Aktivierung der Ruthenium-Katalysatoren im Wasserstoffstrom bei Temperaturen von 180 bis 250°C, bevorzugt bei 190 bis 230°C und Drücken von 1 bis 500 bar, bevorzugt von 20 bis 300 bar innerhalb von 1 bis 20 Stunden, bevorzugt 2 bis 10 Stunden.

Die Ruthenium-Katalysatoren können gegebenenfalls noch weitere Metalle enthalten, wie z.B. Palladium oder Eisen. Der Eisengehalt liegt dabei im allgemeinen im Bereich von 0,5 bis 5 Gew.-%, der Palladium-Gehalt im Bereich von 0,1 bis 5 Gew.-%.

Die Umsetzung führt man bevorzugt kontinuierlich, z.B. in druckfesten Rührbehältern oder in einer Rührbehälterkaskade durch. In einer besonders bevorzugten Ausführungsform werden Rohrreaktoren eingesetzt, in denen das Hydriergut in Sumpf- oder Rieselfahrweise über ein fest angeordnetes Katalysatorbett geleitet wird.

Die beiden Verfahrensstufen können ebenfalls in einem Reaktor durchgeführt werden, in dem Iminierungskatalysatoren und Hydrierkatalysatoren in zwei getrennten Schichten angeordnet sind. In diesem Fall führt man die Iminierung zweckmäßigerweise in Gegenwart von Wasserstoff durch.

Nach der Hydrierung wird überschüssiges Ammoniak gegebenenfalls unter Druck abgetrennt. Die so erhaltenen 2,2-disubstituierten Pentan-1,5-diamine, wie z.B. 2-Methyl-2-propyl-pentan-1,5-diamin aus 4-Cyano-2-methyl-2-propyl-butanal oder 2-n-Butyl-2-ethyl-pentan-1,5-diamin aus 4-Cyano-2-butyl-2-ethyl-butanal, lassen sich durch fraktionierende Destillation isolieren. 3-substituierte Piperidine, wie z.B. 3,3-Dimethyl-piperidin aus 4-Cyano-2-methyl-2-propyl-butanal oder 3-Butyl-3-ethyl-piperidin aus 4-Cyano-2-butyl-2-ethyl-butanal, entstehen nur in untergeordnetem Maße als Nebenprodukt.

Die Ausgangsstoffe für die Herstellung der 2,2-disubstituierten Pentan-1,5-diamine, die 2,2-disubstituierten 4-Cyanobutanale, sind aus 2,2-disubstituierten Aldehyden und Acrylnitril zugänglich.

Nach dem beschriebenen Verfahren, das Gegenstand der DE-A-4010252 ist, werden z.B. insbesondere folgende 2,2-Dialkyl-pentan-1,5-diamine der Formel (IV) hergestellt:
2-Methyl-2-propylpentan-1,5-diamin,
2-Ethyl-2-butylpentan-1,5-diamin,
2-Methyl-2-n-nonylpentan-1,5-diamin,
1-(3-Aminopropyl)-1-aminomethylcyclopentan und
1-(3-Aminopropyl)-1-aminomethylcyclohexan
Zur Herstellung der 2,2-Dialkyl-pentan-1,5-dicarbamidsäurechloride der Formel (III) können die 2,2-Dialkyl-pentan-1,5-diamine direkt oder als Salze, vorzugsweise als Hydrochloride in Lösungs- oder Verdünnungsmitteln nach bekannten Methoden phosgeniert werden. Geeignete Lösungsmittel sind beispielsweise Toluol, Xylol, Chlorbenzol, Dichlorbenzol oder Mono- und/oder Dicarbonsäureester mit Siedepunkten von 165 bis 250°C, wie z.B. Benzoesäuremethylester, Oxalsäure- und/oder Adipinsäuredimethylester. Eine Lösung der 2,2-Dialkyl-pentan-1,5-diamine oder eine Suspension der entsprechenden Salze wird danach bei Temperaturen von ungefähr 0 bis 80°C, vorzugsweise 10 bis 50°C mit 1 bis 6 Mol, vorzugsweise 1 bis 2,5 Mol, und insbesondere 1 bis 1,5 Mol Phosgen pro -NH₂- oder -NH₂·HCl-Gruppe zur Reaktion gebracht. Das gasförmige oder flüssige Phosgen wird hierbei der Reaktionsmischung mit einer solchen Geschwindigkeit zugeführt, daß die austretenden Gase überwiegend aus Chlorwasserstoff bestehen. Durch Abtrennung des Lösungsmittels, beispielsweise durch Destillation unter Normal- oder vermindertem Druck können die 2,2-Dialkyl-pentan-1,5-dicarbamidsäurechloride isoliert und nach bekannten Methoden gereinigt werden.

Vorzugsweise werden jedoch die gebildeten 2,2-Dialkyl-pentan-1,5-dicarbamidsäurechloride ohne intermediäre Isolierung in den beispielhaft genannten Lösungsmitteln bei Temperaturen von 80 bis 200°C, vorzugsweise von 120 bis 180°C in die erfindungsgemäßen 2,2-Dialkyl-pentan-1,5-diisocyanate der Formel (I) und Chlorwasserstoff gespalten.

Nach beendeter Phosgenierung und Spaltung wird das Lösungsmittel, vorzugsweise unter vermindertem Druck, beispielsweise von 100 bis 5 mbar abdestilliert. Gegebenenfalls kann es auch vorteilhaft sein, den Chlorwasserstoff und evtl. vorliegendes überschüssiges Phosgen mit Hilfe von Stickstoff oder einem anderen Inertgas aus der Diisocyanatlösung auszutreiben, bevor das Lösungsmittel abdestilliert wird.

Die erhaltenen rohen 2,2-Dialkyl-pentan-1,5-diisocyanate können nach bekannten Methoden, beispielsweise durch Destillation unter vermindertem Druck, gereinigt werden.

Nach bevorzugten Ausführungsformen werden die erfindungsgemäßen 2,2-Dialkyl-pentan-1,5-diurethane der Formel (II) hergestellt durch Umsetzung von 2,2-Dialkyl-pentan-1,5-diaminen der Formel (IV) mit
a) Harnstoff und einem primären und/oder sekundären Alkohol R³OH bzw. R⁴OH oder vorzugsweise
b) Harnstoff und einem primären und/oder sekundären Alkohol R³OH bzw. R⁴OH in Gegenwart von Carbamidsäurealkylestern und/oder Dialkylcarbonaten und
gegebenenfalls Abtrennung des gebildeten Ammoniaks. Die Reaktionen können in Gegenwart oder Abwesenheit von Katalysatoren durchgeführt werden.

Die Diurethane nach Formel II können jedoch auch nach anderen Methoden erhalten werden, wie z.B. durch Umsetzung der Diamine mit Carbamidsäurealkylester nach Angaben der EP-A-18 588 oder mit Dialkylcarbonaten, zweckmäßigerweise in Gegenwart von Alkoholen oder durch Umsetzung der Diamine mit Chlorkohlensäurealkylestern.

Nach den bevorzugt durchgeführten Verfahren zur Herstellung der 2,2-Dialkyl-pentan-1,5-diurethane der Formel II werden die 2,2-Dialkyl-pentan-1,5-diamine der Formel IV mit Harnstoff und Alkohol im Molverhältnis 1:1,5 bis 10:2 bis 50, vorzugsweise 1:2,0 bis 2,5:4 bis 20 und insbesondere 1:2,0 bis 2,3:4 bis 10 in Abwesenheit oder in Gegenwart von Katalysatoren bei Reaktionstemperaturen von 175 bis 250°C, vorzugsweise 180 bis 230°C zur Reaktion gebracht. Der im Laufe der Reaktion gebildete Ammoniak wird zweckmäßigerweise sofort abgetrennt. Die Reaktion wird vor allem bei Verwendung niedrig siedender Alkohole unter Druck durchgeführt, wobei der Druck so eingestellt wird, daß das Reaktionsgemisch bei der angewandten Reaktionstemperatur siedet. In Abhängigkeit von dem verwendeten Alkohol beträgt der Druck üblicherweise 0,1 bis 60 bar, vorzugsweise 1 bis 40 bar. Für diese Reaktionsbedingungen ergeben sich Reaktionszeiten von 0,5 bis 50, vorzugsweise 3 bis 15 Stunden.

Als Alkohole R³OH und R⁴OH, die gleich oder verschieden sein können, eignen sich prinzipiell beliebige, gegebenenfalls substituierte primäre und/oder sekundäre aliphatische und/oder cycloaliphatische Alkohole. Vorzugsweise wird man jedoch solche auswählen, deren Siedepunkte genügend weit vom Siedepunkt des durch nachfolgende chemische Spaltung erhaltenen 2,2-Dialkyl-pentan-1,5-diisocyanats entfernt liegen, so daß einerseits eine möglichst quantitative Trennung der Spaltprodukte Diisocyanat und Alkohol möglich ist und andererseits die gebildeten 2,2-Dialkyl-pentan-1,5-diurethane, möglichst unzersetzt verdampft werden können.

Als Alkohole R³OH bzw. R⁴OH in Betracht kommen beispielsweise aliphatische, gegebenenfalls substituierte primäre Alkohole mit 1 bis 20 Kohlenstoffatomen, vorzugsweise 1 bis 10 Kohlenstoffatomen oder insbesondere 3 bis 6 Kohlenstoffatomen in einem linearen Alkylrest, sekundäre Alkohole mit 3 bis 20 Kohlenstoffatomen, vorzugsweise 3 bis 10 Kohlenstoffatomen und insbesondere 3 bis 6 Kohlenstoffatomen in einem verzweigten Alkylrest und/oder cycloaliphatische, gegebenenfalls substituierte Alkohole mit 5 bis 12 Kohlenstoffatomen und insbesondere 5 bis 8 Kohlenstoffatomen im gegebenenfalls substituierten Cycloakylrest. Beispielhaft genannt seien Alkohole wie Methanol, Ethanol, Propanol, n-Butanol, Isobutanol, 2- und 3-Methylbutanol, Neopentylalkohol, Pentanol, 2-Methyl-pentanol, n-Hexanol, 2-Ethylhexanol, n-Heptanol, n-Octanol, n-Nonanol, n-Decanol, n-Dodecanol, Isopropanol, sec.Butanol, sec.-Isoamylalkohol, Cyclopentanol, Cyclohexanol, 2-, 3- oder 4-Methyl-cyclohexanol und tert.-Butyl-cyclohexanol. Vorzugsweise verwendet werden Methanol, Ethanol, n-Propanol, n-Butanol, iso-Butanol, n-Pentanol, iso-Pentanol, n-Hexanol oder Gemische der aliphatischen und/oder cycloaliphatischen Alkohole sowie insbesondere n-Propanol, n- und/oder iso-Butanol.

Wie bereits dargelegt wurde, kann die Umsetzung der 2,2-Dialkyl-pentan-1,5-diamine der Formel IV mit Harnstoff und Alkohol auch in Gegenwart von Carbamidsäurealkylester und/oder Dialkylcarbonaten durchgeführt werden. Bei diesen Verfahrensvarianten werden das Dialkylcarbonat zweckmäßigerweise in einer Menge von 1 bis 30 Mol.%, vorzugsweise 5 bis 25 Mol.% oder die Carbamidsäurealkylester in einer Menge von 1 bis 20 Mol.%, vorzugsweise von 5 bis 18 Mol.%, bezogen auf die 2,2-Dialkylpentan-1,5-diamine eingesetzt. Vorzugsweise verwendet werden jedoch Mischungen aus Dialkylcarbonaten und Carbamidsäurealkylestern in den genannten Mengenverhältnissen. Als Dialkylcarbonate und/oder Carbamidsäurealkylester setzt man bevorzugt solche ein, deren Alkylreste dem Alkylrest des verwendeten Alkohols entsprechen.

Zur Erhöhung der Reaktionsgeschwindigkeit können die 2,2-Dialkylpentan-1,5-diurethane der Formel (II) in Gegenwart von Katalysatoren hergestellt werden. Diese werden zweckmäßigerweise in Mengen von 0,1 bis 20 Gew.%, vorzugsweise 0,5 bis 10 Gew.%, und insbesondere 1 bis 5 Gew.%, bezogen auf das Gewicht der 2,2-Dialkyl-pentan-1,5-diamine verwendet. Als Katalysatoren eignen sich anorganische oder organische Verbindungen, die ein oder mehrere Kationen, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14th. Edition, publiziert von Chemical Rubber Publishing Co., 23 Superior Ave. N.E., Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxilate, Chelate, Carbonate und Thio- oder Dithiocarbamate. Beispielhaft genannt seien die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen, Cobalt und Nickel. Vorzugsweise Verwendung finden die Kationen von Lithium, Calcium, Aluminium, Zinn, Bismut, Antimon, Kupfer, Zink, Titan, Vanadium, Chrom, Molybdän, Mangan, Eisen und Cobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminium-isobutylat, Aluminiumtrichlorid, Bismut-(III)-chlorid, Kupfer-(II)-acetat, Kupfer-(II)-sulfat, Kupfer-(II)-nitrat, Bis-(triphenylphosphinoxido)kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylat, Cer-(IV)-oxid, Uranylacetat, Titantetrabutanolat, Titantetrachlorid, Titantetraphenolat, Titannaphthenat, Vanadium-(III)-chlorid, Vanadiumacetonylacetat, Chrom-(III)-chlorid, Molybdän-(VI)-oxid, Molybdänacetylacetonat, Wolfram-(VI)-oxid, Mangen-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Cobaltacetat, Cobaltchlorid, Cobaltsulfat, Cobaltnaphthenat, Nickelchlorid, Nickelacetat und Nickelnaphthenat sowie deren Gemische.

Es hat sich als vorteilhaft erwiesen, das entstehende Ammoniak sofort aus der Reaktionsmischung, beispielsweise durch Destillation, abzutrennen. Die hierfür verwendete Vorrichtung, beispielsweise eine Destillationskolonne, wird bei Temperaturen von 60 bis 150°C, vorzugsweise 65 bis 120°C betrieben, so daß eine Belegung durch Ammoniumcarbaminat, welches in minimalen Mengen aus Ammoniak und Kohlendioxid durch Zersetzung von Harnstoff gebildet wird, vermieden werden kann.

Nach beendeter Reaktion werden aus der erhaltenen Reaktionsmischung zweckmäßigerweise der Alkohol, die Dialkylcarbonate und/oder Carbamidsäurealkylester abgetrennt und zur Wiederverwendung bei nachfolgenden Ansätzen bereitgehalten; bei kontinuierlicher Fahrweise werden sie jedoch vorzugsweise direkt an den Anfang des Diurethanherstellungsprozesses zurückgeführt.

Die Abtrennung der genannten Verbindungen kann ein- oder mehrstufig durchgeführt werden. Vorzugsweise findet ein zweistufiges Verfahren Anwendung. Hierbei wird in der 1. Stufe der Alkohol bis auf einen Restalkoholgehalt von 1 bis 30 Gew.%, vorzugsweise 2 bis 15 Gew.%, bezogen auf das Gewicht der Reaktionsmischung abdestilliert und an den Prozeßbeginn zurückgeführt.

Die aufkonzentrierte Reaktionsmischung, die überwiegend aus 2,2-Dialkylpentan-1,5-diurethanen und gegebenenfalls -oligoharnstoff-polyurethanen besteht und noch den restlichen Alkohol, Dialkylcarbonat und/ oder Carbamidsäurealkylester enthält, wird in der zweiten Stufe in einer Strippkolonne mit 50 bis 5000 Liter, vorzugsweise 100 bis 1000 Liter Inertgas pro Liter aufkonzentrierter Reaktionsmischung und Stunde bei Stripptemperaturen von 50 bis 200°C, vorzugsweise 120 bis 180°C behandelt, um den restlichen Alkohol, die Dialkylcarbonate und/oder Carbamidsäurealkylester praktisch vollständig abzutrennen. Geeignete Inertgase hierfür sind beispielsweise Stickstoff, Kohlenmonoxid, Edelgase und Erdgase. Die abgestrippten leichtersiedenden Verbindungen werden kondensiert, gegebenenfalls zwischengelagert und zur Wiederverwendung bei weiteren Ansätzen bereitgehalten. Bei kontinuierlicher Fahrweise werden sie vorzugsweise an den Anfang des Diurethanherstellungsverfahrens zurückgeführt.

Aus der nach der Destillation oder vorzugsweise Strippung erhaltenen Reaktionsmischung, die im wesentlichen aus 2,2-Dialkyl-pentan-1,5-diurethanen der Formel II und gegebenenfalls 2,2-Dialkyl-pentan-oligoharnstoffpolyurethanen besteht, können nach bekannten Methoden, beispielsweise durch Destillation, die 2,2-Dialkyl-pentan-1,5-diurethane isoliert und gegebenenfalls einem zusätzlichen Reinigungsprozeß unterworfen werden.

Die im wesentlichen aus 2,2-Dialkyl-pentan-1,5-diurethanen und gegebenenfalls -oligoharnstoff-polyurethanen bestehende Reaktionsmischung wird jedoch vorzugsweise direkt thermisch in 2,2-Dialkyl-pentan-1,5-diisocyanate der Formel I und Alkohol gespalten.

Die thermische Spaltung kann in an sich bekannter Weise in der Gasphase bei Temperaturen von über 300°C unter vermindertem Druck, beispielsweise in Abwesenheit gelöster Katalysatoren gemäß DE-A-24 10 505 (US 3 870 739) oder in Gegenwart von Katalysatoren, beispielsweise analog den Angaben der DE-A-19 44 719 (GB 1 247 451) oder in flüssiger Phase bei Temperaturen von 175 bis 350°C, vorzugsweise 200 bis 280°C, beispielsweise in Gegenwart von Lösungsmitteln katalysatorfrei analog den Angaben der DE-A-24 21 503 (US 3 962 302) oder der DE-A-25 30 001 (US 3 919 280) oder in Gegenwart von Lösungsmitteln und Katalysatoren, beispielsweise analog den Angaben der DE-A-26 35 490 durchgeführt werden.

Die gegebenenfalls in untergeordneten Mengen 2,2-Dialkyl-pentan-oligoharnstoff-polyurethane enthaltende 2,2-Dialkyl-pentan-1,5-diurethanmischung kann in flüssiger oder in fester Form oder auch als Suspension oder Lösung in einem unter den Reaktionsbedingungen inerten Lösungsmittel in einem Verdampfer verdampft und einem nachfolgenden Spaltreaktor thermisch gespalten werden.

Nach der bevorzugten Ausführungsform des erfindungsgemäßen Verfahrens wird die Diurethanmischung lösungsmittelfrei, in Form einer auf 80 bis 180°C, vorzugsweise 100 bis 150°C erhitzten Schmelze mit einer Dosierpumpe in den Verdampfer eingebracht.

Als Verdampfer, die bei Temperaturen von 200 bis 300°C, vorzugsweise von 220 bis 300°C und insbesondere von 240 bis 280°C und unter einem Druck von 0,1 bis 200 mbar, vorzugsweise von 5 bis 100 mbar betrieben werden, haben sich insbesondere Dünnschichtverdampfer oder Wirbelschichtverdampfer bewährt. Es können jedoch auch beliebige andere Verdampfer verwendet werden, z.B. Schneckenverdampfer, A.P.-Reaktoren (Hersteller: Krauss-Maffei), Metallschlangen- oder Rührbett-Verdampfer.

Bei Verwendung von Dünnschichtverdampfern ist es zwar durch eine ausreichende Wärmezufuhr möglich, die gesamte zugeführte 2,2-Dialkylpentan-1,5-diurethanmischung zu verdampfen. Vorteilhafterweise wird jedoch ein Teil der zugeführten Diurethanmischung gemeinsam mit gegebenenfalls vorhandenem 2,2-Dialkyl-pentan-oligoharnstoff-polyurethan unverdampft als Schmelze aus dem Verdampfer ausgeschleust, da man hierdurch einen bedeutenden Reinigungseffekt an der Verdampferwand erzielt. Das Gewichtsverhältnis von verdampftem zu unverdampften 2,2-Dialkyl-pentan-1,5-diurethanen kann in breiten Grenzen, beispielsweise von 20:80 bis 90:10 variiert werden. Die aus dem Verdampfer ausgeschleuste Schmelze wird vorzugsweise direkt an den Anfang des Diurethanherstellungsprozesses, die Diurethanisierungsstufe, zurückgeführt.

Die 2,2-Dialkyl-pentan-1,5-diurethan-Dämpfe werden in den Spaltreaktor eingebracht und bei einer Temperatur von über 300°C, vorzugsweise 310 bis 480°C und insbesondere 350 bis 450°C und unter vermindertem Druck, beispielsweise von 0,1 bis 200 mbar, vorzugsweise 0,1 bis 100 mbar und insbesondere 1 bis 50 mbar, diskontinuierlich oder vorzugsweise kontinuierlich in 2,2-Dialkyl-pentan-1,5-diisocyanate der Formel (I) und Alkohol thermisch gespalten.

Der Spaltreaktor, der im allgemeinen säulenförmig ist, kann einen Querschnitt in beliebiger Form aufweisen. Vorzugsweise verwendet man langgestreckte, zylinderförmige Spaltreaktoren. Das Verhältnis von Innendurchmesser zu Länge des Spaltreaktors beträgt im allgemeinen 1:2 bis 1:1000, vorzugsweise 1:10 bis 1:500. Die Spaltreaktoren können senkrecht oder waagrecht ausgerichtet sein und auch Zwischenanlagen einnehmen. Vorzugsweise verwendet werden Röhrenöfen als Spaltreaktoren, bei denen der Rohrinnendurchmesser etwa 10 bis 100 mm und die Rohrlänge ungefähr 0,5 bis 5 m beträgt.

Zweckmäßigerweise führt man die Spaltung in Gegenwart von thermisch stabilen Reaktorfüllkörpern durch. Als Füllkörper geeignet sind alle temperaturbeständigen und gasdurchlässigen Materialien, wie z.B. Perlen, Wolle, Ringe und/oder Späne aus Kohle, Stahl, Messing, Kupfer, Zink, Aluminium, Titan, Chrom, Cobalt, Nickel und/oder Quarz. Einige dieser Materialien wie Stahl, Messing, Aluminium und Zink, haben sich besonders bewährt und werden daher bevorzugt verwendet, da sie zu besseren Spaltergebnissen führen. Hierbei ist noch ungeklärt, ob es sich um katalytische oder physikalische Effekte, wie beispielsweise eine bessere Wärmeübertragung, handelt ober ob eine synergistische Kombination beider Effekte vorliegt.

Aus dem Spaltreaktor führt man die in der Dampfphase befindlichen Dissoziationsprodukte, die nahezu ausschließlich aus 2,2-Dialkylpentan-1,5-diisocyanat und Alkohol bestehen, in eine mehrstufige, vorzugsweise zweistufige Dampfkondensationsvorrichtung. In der ersten Kondensationsstufe, die abhängig vom Systemdruck von 0,1 bis 100 mbar bei Temperaturen von 60 bis 120°C betrieben wird, kondensieren die 2,2-Dialkyl-pentan-1,5-diisocyanate nahezu vollständig aus.

In der zweiten Kondensationsstufe wird im wesentlichen Alkohol kondensiert, der zur Diurethanherstellung zurückgeführt wird. Die Temperatur der zweiten Kondensationsstufe richtet sich nach dem Siedepunkt des zu kondensierenden Alkohols und dem Systemdruck und beträgt beispielsweise 5 bis 30°C.

Die in der ersten Kondensationsstufe erhaltenen 2,2-Dialkyl-pentan-1,5-diisocyanate der Formel I werden üblicherweise einer Reindestillation unterworfen und besitzen danach eine Reinheit von über 99,5 Gew.%. Das hierbei anfallende Sumpfprodukt kann gegebenenfalls an den Anfang der Diurethanherstellung zurückgeführt werden.

Je nach Wahl der Kondensationstemperaturen und in Abhängigkeit vom Systemdruck, können in der ersten Kondensationsstufe Alkohol und in der zweiten Kondensationsstufe Diisocyanate in unterschiedlichen Mengen mitkondensiert werden. Nach einer bevorzugten Ausführungsform läßt man in der zweiten Kondensationsstufe mitkondensiertes Diisocyanat mit überschüssigem Alkohol zu 2,2-Dialkyl-pentan-1,5-diurethanen abreagieren und führt es nach dem Abtrennen von Alkohol erneut der Verdampfung und Spaltung zu. Es ist jedoch auch möglich nach einer ebenfalls bevorzugten Ausführungsform, die Diurethane mit dem Dialkylcarbonat und/oder Carbamidsäurealkylester an den Anfang des Diurethanherstellungsverfahren zurückzuführen.

Auf analoge Weise läßt man in der ersten Kondensationsstufe mitkondensierten Alkohol mit überschüssigem Diisocyanat abreagieren und führt die Reaktionsprodukte nach der destillativen Abtrennung des Diisocyanates der Verdampfung und Spaltung zu oder in der bevorzugten Ausführungsform unter Vermischung mit dem in der zweiten Kondensationsstufe erhaltenen Alkohol an den Beginn des Diurethanherstellungsverfahrens zurück.

Die neuen 2,2-Dialkylpentan-, 2,2-Dialkenylpentan- und 2-Alkyl-2-alkenylpentan-1,5-diisocyanate und 1-(3-Isocyanatopropyl)-1-isocyanatomethyl-cycloalkane der Formel I sind wertvolle Ausgangsstoffe zur Herstellung von Polyurethan-, Polyharnstoff-, Polyurethan-Polyharnstoff-Kunststoffen, beispielsweise Lacken, überzugs-, Dichtungsmassen, Klebstoffen, Elastomeren, Fasern, Bodenbelägen, Schaumstoffen u.a. nach dem Polyisocyanat-polyadditionsverfahren. Durch die Auswahl der Reste R¹ und R² können die physikalischen Eigenschaften wie z.B. Siedepunkt, Dampfdruck, Polarität, Löslichkeit modifiziert und den örtlichen Verarbeitungsbedingungen angepaßt und gegebenenfalls die mechanischen Eigenschaften der erhaltenen Kunststoffe eingestellt, variiert und verbessert werden. Die 2,2-Dialkenylpentan- und 2-Alkyl-2-alkenylpentan-1,5-diisocyanate besitzen außer den NCO-Gruppen zusätzlich mindestens einen olefinisch ungesättigten reaktiven Rest und sind daher Folgereaktionen, insbesondere Polymerisationsreaktionen, zugänglich. Die Diisocyanate eignen sich insbesondere zur Herstellung von lichtbeständigen Polyurethanlacken und -überzügen.

Die neuen 2,2-Dialkylpentan-, 2,2-Dialkenylpentan- und 2-Alkyl-2-alkenylpentan-1,5-diurethane und 1-(3-N-carbalkoxy-aminopropyl)-1-N-carbalkoxymethyl-cycloalkane der Formel II sind wertvolle End- und Zwischenprodukte.

Sie können beispielsweise als Schädlingsbekämpfungsmittel eingesetzt werden. Als Zwischenprodukte werden sie als Aufbaukomponente für Polykondensationssysteme, beispielsweise durch Reaktion mit nieder- und/oder höhermolekularen Polyhydroxylverbindungen und/oder Polyaminen zur Herstellung von Kunststoffen oder -fasern oder als olefinisch ungesättigtes Monomer verwendet. Vorzugsweise Anwendung finden sie zur Herstellung von Diisocyanaten durch thermische Spaltung.

Die neuen 2,2-Dialkylpentan-, 2,2-Dialkenylpentan- und 2-Alkyl-2-alkenylpentan-1,5-dicarbamidsäurechloride und 1-(3-N-chlorformyl-aminopropyl)-1-N-chlorformyl-aminomethyl-cycloalkane sind insbesondere geeignet zur Herstellung von Diisocyanaten.

Die Erfindung wird durch die nachstehenden Beispiele zusätzlich erläutert.

### Beispiele

### Beispiel 1

Ein vertikaler Rohrreaktor (Durchmesser: 16 mm, Füllhöhe: 50 cm, ölbeheizter Doppelmantel) wurde mit 81,9 g (93 ml) eines Katalysators mit 3 Gew.-% Ruthenium auf einem Magnesiumoxid/Aluminiumoxid-Träger (10 Gew.-% MgO, 90 Gew.-% Al₂O₃) in Form von 1 bis 1,5 mm Split gefüllt (Katalysatorherstellung durch Porentränkung eines Magnesiumoxid/Aluminiumoxid-Trägers mit wäßriger Rutheniumnitrat-Lösung und Trocknen bei 120°C). Der Katalysator wurde zur Reduktion bei 100 bar unter gleichzeitigem Durchleiten von 100 Nl/h Wasserstoff nach schrittweiser Erhöhung der Temperatur von 100 auf 220°C innerhalb von 6 h 7 h bei 220°C gehalten.

Durch einen vor den Hydrierreaktor geschalteten Rohrreaktor (Durchmesser: 16 mm, Füllhöhe: 50 cm, ölbeheizter Doppelmantel), der mit 63,5 g (100 ml) TiO₂ (Anatas) in Form von 1,5-mm-Strängen gefüllt war, wurde bei einem Druck von 250 bar und einer Temperatur von 60°C stündlich 33,5 g 2-Methyl-2-propyl-4-cyano-butanal (Reinheit: 88,9 %, 29,8 g, 0,195 mol) und 1400 ml (840 g, 49,4 mol) flüssiges Ammoniak gepumpt. Der Austrag wird anschließend bei einem Druck von 250 bar und einer Temperatur von 120°C unter gleichzeitigem Durchleiten von 100 Nl/h (4,5 mol) Wasserstoff von unten nach oben durch den Hydrierreaktor geleitet. Nach Entspannen auf Normaldruck wird NH₃ abdestilliert. Der Austrag aus 37,5 Stunden wird durch fraktionierende Destillation über eine 30 cm Füllkörperkolonne (3 mm Glasringe) aufgetrennt. Man erhält 277,0 g 3-Methyl-3-propyl-piperidin (Kp₂ = 46°C) und 842,1 g 2-Methyl-2-propyl-pentan-1,5-diamin (Kp₂ = 78-81°C). Die Diamin-Ausbeute beträgt 72,9 % d. Th.

### Beispiel 2

Beispiel 1 wird mit 2-Butyl-2-ethyl-4-cyano-butanal als Edukt wiederholt. Dazu wurden durch den Iminierungsreaktor bei einem Druck von 250 bar und einer Temperatur von 60°C stündlich 33,6 g 2-Ethyl-2-butyl-4-cyano-butanal (Reinheit: 89,0 %, 29,9 g, 0,165 mol) und 1344 ml (806 g, 47,4 mol) flüssiges Ammoniak gepumpt. Der Austrag wird anschließend bei einem Druck von 250 bar und einer Temperatur von 120°C unter gleichzeitigem Durchleiten von 100 Nl/h (4,5 mol) Wasserstoff von unten nach oben durch den Hydrierreaktor geleitet. Nach Entspannen auf Normaldruck wird NH₃ abdestilliert. Der Austrag aus 16,7 Stunden wird durch fraktionierende Destillation über eine 30 cm Füllkörperkolonne (3 mm Glasringe) aufgetrennt. Man erhält 166,8 g 3-Butyl-3-ethyl-piperidin (Kp₂ = 73 bis 75°C) und 267,5 g 2-Ethyl-2-butyl-pentan-1,5-diamin (Kp₂ = 105°C). Die Diamin-Ausbeute beträgt 52,1 % d. Th.

### Beispiel 3

In einem 1-l-Rührautoklaven mit aufgesetzter Druckkolonne und Druckregelventil wurden 158 g 2-Methyl-2-propyl-pentan-1,5-diamin, hergestellt nach Beispiel 1, 126 g Harnstoff und 370 g Butanol 4 Stunden bei 230°C und 9 bar unter Abnahme von Ammoniak am Rückfluß gekocht. Man erhielt 590 g einer klaren Flüssigkeit, deren Analyse mittels Gelpermeationschromatographie eine Umwandlung in 2-Methyl-2-propyl-1,5-bis-(butoxicarbonylamino)-pentan von 95 % anzeigte. Nach Abdestillieren von überschüssigem Butanol und geringen Mengen Carbamidsäurebutylester verblieben 355 g einer zähigen Flüssigkeit, die ohne weitere Reinigung in die thermische Spaltung eingesetzt wurde. Durch Säulenchromatographie an Kieselgel erhielt man reines 2-Methyl-2-propyl-1,5-bis-(butoxi-carbonylamino)-pentan als farbloses, zähes Öl.

| C,H,N-Analyse: | | | |
|---|---|---|---|
| | C | H | N |
| berechnet | 63,65 | 10,68 | 7,81 |
| gefunden | 63,68 | 10,72 | 7,78 |

### Beispiel 4

Eine Spaltapparatur, bestehend aus einem Dünnschichtverdampfer, einem Spaltreaktor (zylindrisches Rohr aus V2A-Stahl von ca. 1 l Leervolumen bestückt mit verzinkten Blechfüllkörpern) und einer zweistufigen Dampfkondensationsvorrichtung, wurde auf 5 mbar evakuiert. Innerhalb von 2 Stunden wurden 350 g des nach Beispiel 3 erhaltenen 2-Methyl-2-propyl-1,5-bis(butoxicarbonylamino)-pentans in den auf 260°C erhitzten Dünnschichtverdampfer eingebracht, wovon 315 g verdampften und 35 g abliefen. Die Diurethandämpfe gelangten in den Spaltreaktor, dessen durchschnittliche Temperatur 400°C betrug. Die austretenden Spaltgase wurden in einer nachgeschalteten zweistufigen Kondensationsvorrichtung bei 65 bis 18°C fraktionierend kondensiert. Im ersten Kondensator fielen 205 g rohes Diisocyanat an, das durch Vakuumdestillation (Übergangstemperatur 105 bis 107°C/0,5 mbar) gereinigt wurde. Man erhielt 157,3 g (53 % Ausbeute) 2-Methyl-2-propyl-pentan-1,5-diisocyanat mit einer Reinheit von 98 %.

### Beispiel 5

In einem 1-l-Rührautoklaven mit aufgesetzter Druckkolonne und Druckregelventil wurden 186 g 2-Ethyl-2-butyl-pentan-1,5-diamin, hergestellt nach Beispiel 2, 126 g Harnstoff und 444 g Butanol 5 Stunden bei 230°C und 10 bar unter Abnahme von Ammoniak am Rückfluß gekocht. Man erhielt 670 g einer klaren Flüssigkeit, deren Analyse mittels Gelpermeationschromatographie eine Umwandlung in 2-Ethyl-4-butyl-1,5-bis(butoxicarbonylamino)-pentan von 98 % anzeigte. Nach Abdestillieren von überschüssigem Butanol und geringen Mengen Carbamidsäurebutylester verblieben 390 g einer zähen Flüssigkeit, die ohne weitere Reinigung in die thermische Spaltung eingesetzt wurde. Durch Säulenchromatographie an Kieselgel erhielt man reines 2-Ethyl-2-butyl-1,5-bis(butoxicarbonylamino)-pentan als farbloses, zähes Öl.

| C,H,N-Analyse: | | | |
|---|---|---|---|
| | C | H | N |
| berechnet | 65,25 | 10,95 | 7,25 |
| gefunden | 65,27 | 10,99 | 7,20 |

### Beispiel 6

Die Spaltung wurde in der in Beispiel 4 beschriebenen Apparatur durchgeführt. 550 g des nach Beispiel 5 erhaltenen 2-Ethyl-2-butyl-1,5-bis(butoxicarbonylamino)-pentans wurden innerhalb von 3 Stunden in den auf 280°C beheizten und auf 8 bis 9 mbar evakuierten Dünnschichtverdampfer eingebracht, wobei 502 g verdampften und 48 g abliefen. Die Temperatur im Spaltreaktor betrug durchschnittlich 400°C. Im bei 85°C betriebenen Kondensator wurden 340 g rohes Diisocyanat gesammelt. Dieses wurde durch Destillation über einen Dünnschichtverdampfer (Öltemperatur 160°C/ 0,2 mbar) vorgereinigt und nochmals unter vermindertem Druck bei 113°C/0,5 mbar destilliert. Man erhielt 223 g (70 % Ausbeute) 2-Ethyl-2-butyl-pentan-1,5-diisocyanat mit einer Reinheit von 99 %.

### Beispiel 7

Zu einer auf 0°C abgekühlten Mischung aus 200 g o-Dichlorbenzol und 60 g Phosgen tropfte man unter kräftigem Rühren und Eiskühlung 15,8 g 2-Methyl-2-propyl-pentan-1,5-diamin. Nach beendeter Zugabe wurde die entstandene Suspension auf 130°C erwärmt und bei dieser Temperatur 2,5 Stunden Phosgen durch das Reaktionsgemisch geleitet. Nach dem Abkühlen wurde das überschüssige Phosgen mit einem kräftigen Stickstoffstrom ausgetrieben, das o-Dichlorbenzol unter vermindertem Druck bei 10 mbar abdestilliert und der Rückstand bei 107 bis 110°C bei 0,5 mbar destilliert. Man erhielt 15,2 g (73 % Ausbeute) 2-Methyl-2-propyl-pentan-1,5-diisocyanat.

## Patentansprüche

1. 2,2-Dialkylpentan-1,5-diisocyanate der Formel (I) 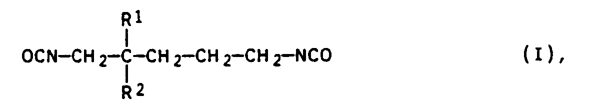 in der R¹ und R² gleich oder verschieden sind und bedeuten:
einen linearen Alkylrest mit 1 bis 12 Kohlenstoffatomen,
einen verzweigten Alkylrest mit 3 bis 12 Kohlenstoffatomen,
einen linearen Alkenylrest mit 2 bis 12 Kohlenstoffatomen oder
einen verzweigten Alkenylrest mit 4 bis 12 Kohlenstoffatomen, wobei das
2,2-Dimethylpentan-1.5-diisocyanat ausgenommen ist.

2. 2,2-Dialkylpentan-1,5-diurethane der Formel (II) 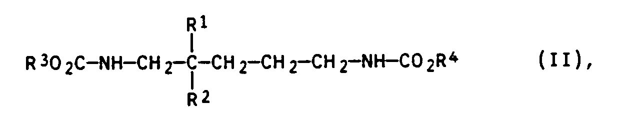 in der R¹ und R² gleich oder verschieden sind und bedeuten:
einen linearen Alkylrest mit 1 bis 12 Kohlenstoffatomen,
einen verzweigten Alkylrest mit 3 bis 12 Kohlenstoffatomen,
einen linearen Alkenylrest mit 2 bis 12 Kohlenstoffatomen,
einen verzweigten Alkenylrest mit 4 bis 12 Kohlenstoffatomen,
die Reste R¹ und R² gemeinsam eine Alkylengruppe mit 4 bis 7 Kohlenstoffatomen oder
eine Alkylengruppe mit 4 bis 7 Kohlenstoffatomen, die mit einer bis 5 C₁- bis C₄-Alkylgruppen substituiert ist
und
R³ und R⁴ gleich oder verschieden sind und bedeuten:
einen linearen Alkylrest mit 1 bis 20 Kohlenstoffatomen,
einen verzweigten Alkylrest mit 3 bis 20 Kohlenstoffatomen oder
einen Cycloalkylrest mit 5 bis 12 Kohlenstoffatomen.

3. 2,2-Dialkylpentan-1,5-dicarbamidsäurechloride der Formel (III) 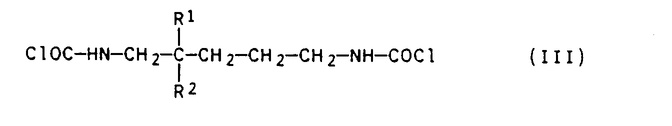 in der R¹ und R² gleich oder verschieden sind und bedeuten:
einen linearen Alkylrest mit 1 bis 12 Kohlenstoffatomen,
einen verzweigten Alkylrest mit 3 bis 12 Kohlenstoffatomen,
einen linearen Alkenylrest mit 2 bis 12 Kohlenstoffatomen oder
einen verzweigten Alkenylrest mit 4 bis 12 Kohlenstoffatomen, wobei das
2,2-Dimethylpentan-1.5-dicarbamidsäurechlorid ausgenommen ist.

4. Verfahren zur Herstellung von 2,2-Dialkylpentan-1,5-diisocyanaten der Formel (I), dadurch gekennzeichnet, daß man 2,2-Dialkylpentan-1,5-diurethane der Formel (II) in Gegenwart oder Abwesenheit von Katalysatoren
a) in der Gasphase bei Temperaturen von über 300°C unter vermindertem Druck oder
b) in flüssiger Phase bei Temperaturen von 175 bis 350°C
thermisch spaltet.

5. Verfahren zur Herstellung von 2,2-Dialkylpentan-1,5-diurethanen der Formel (II), dadurch gekennzeichnet, daß man 2,2-Dialkylpentan-1,5-diamine der Formel (IV) 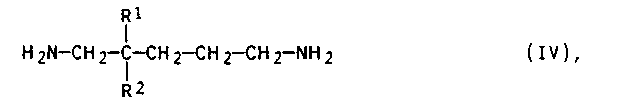 in der R¹ und R² gleich oder verschieden sind und bedeuten:
einen linearen Alkylrest mit 1 bis 12 Kohlenstoffatomen,
einen verzweigten Alkylrest mit 3 bis 12 Kohlenstoffatomen,
einen linearen Alkenylrest mit 2 bis 12 Kohlenstoffatomen,
einen verzweigten Alkenylrest mit 4 bis 12 Kohlenstoffatomen,
die Reste R¹ und R² gemeinsam eine Alkylengruppe mit 4 bis 7 Kohlenstoffatomen oder
eine Alkylengruppe mit 4 bis 7 Kohlenstoffatomen, die mit einer bis 5 C₁- bis C₄-Alkylgruppen substituiert ist,
in Gegenwart oder Abwesenheit von Katalysatoren mit
a) Harnstoff und einem primären und/oder sekundären Alkohol R³OH und/oder R⁴OH umsetzt oder mit
b) Harnstoff und einem primären und/oder sekundären Alkohol R³OH und/oder R⁴OH, in Gegenwart von Carbamidsäurealkylestern und/oder Dialkylcarbonaten umsetzt, wobei
R³ und R⁴ gleich oder verschieden sind und bedeuten:
einen linearen Alkylrest mit 1 bis 20 Kohlenstoffatomen,
einen verzweigten Alkylrest mit 3 bis 20 Kohlenstoffatomen oder
einen Cycloalkylrest mit 5 bis 12 Kohlenstoffatomen,
und den entstehenden Ammoniak gegebenenfalls abtrennt.

6. Verfahren zur Herstellung von 2,2-Dialkyl-pentan-1,5-diurethanen der Formel (II) nach Anspruch 5, dadurch gekennzeichnet, daß man die 2,2-Dialkyl-pentan-1,5-diamine (IV) mit Harnstoff und Alkohol im Molverhältnis 1:1,5 bis 10:2 bis 50 umsetzt.

7. Verfahren zur Herstellung von 2,2-Dialkyl-pentan-1,5-diurethanen der Formel (II) nach Anspruch 5, dadurch gekennzeichnet, daß man den dem Alkohol entsprechenden Carbamidsäurealkylester in einer Menge von 1 bis 20 Mol.%, bezogen auf das 2,2-Dialkyl-pentan-1,5-diamin (IV), verwendet.

8. Verwendung von 2,2-Dialkyl-pentan-1,5-diurethane der Formel (II) zur Herstellung von 2,2-Dialkylpentan-1,5-diisocyanaten der Formel (I).

9. Verwendung von 2,2-Dialkylpentan-diisocyanaten nach Anspruch 1, mit Ausnahme von 2,2-Dimethylpentan-diisocyanat, zur Herstellung von Klebstoffen, Dichtungsmassen, Überzugsmitteln und kompakten oder zelligen Kunststoffen nach dem Polyisocyanat-polyadditionsverfahren.

## Claims

1. A 2,2-dialkylpentane 1,5-diisocyanate of the formula (I) 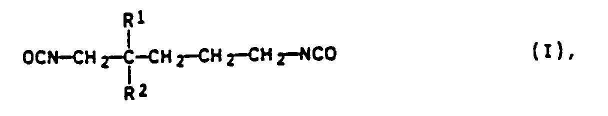 where R¹ and R² are identical or different and are each linear alkyl of 1 to 12 carbon atoms, branched alkyl of 3 to 12 carbon atoms, linear alkenyl of 2 to 12 carbon atoms or branched alkenyl of 4 to 12 carbon atoms, with the exception of 2,2-dimethylpentane 1,5-diisocyanate.

2. A 2,2-dialkylpentane-1,5-diurethane of the formula (II) 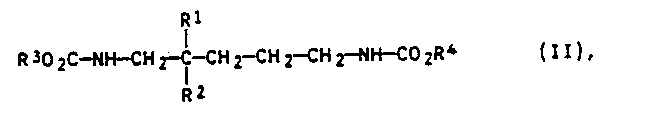 where R¹ and R² are identical or different and are each linear alkyl of 1 to 12 carbon atoms, branched alkyl of 3 to 12 carbon atoms, linear alkenyl of 2 to 12 carbon atoms or branched alkenyl of 4 to 12 carbon atoms, R¹ and R² together are alkylene of 4 to 7 carbon atoms which is unsubstituted or monosubstituted to pentasubstituted by C₁-C₄-alkyl and R³ and R⁴ are identical or different and are each linear alkyl of 1 to 20 carbon atoms, branched alkyl of 3 to 20 carbon atoms or cycloalkyl of 5 to 12 carbon atoms.

3. A 2,2-dialkylpentane-1,5-dicarbamoyl chloride of the formula (III) 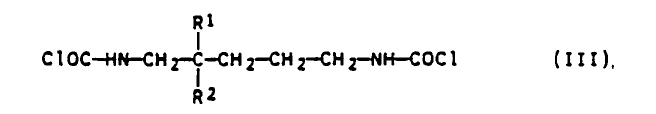 where R¹ and R² are identical or different and are each linear alkyl of 1 to 12 carbon atoms, branched alkyl of 3 to 12 carbon atoms, linear alkenyl of 2 to 12 carbon atoms or branched alkenyl of 4 to 12 carbon atoms, with the exception of 2,2-dimethylpentane-1,5-dicarbamoyl chloride.

4. A process for the preparation of a 2,2-dialkylpentane 1,5-diisocyanate of the formula (I), wherein a 2,2-dialkylpentane-1,5-diurethane of the formula (II) is subjected to thermal cleavage in the presence or absence of a catalyst
a) in the gas phase at above 300°C under reduced pressure or
b) in the liquid phase at from 175 to 350°C.

5. A process for the preparation of a 2,2-dialkylpentane-1,5-diurethane of the formula (II), wherein a 2,2-dialkylpentylene-1,5-diamine of the formula (IV) 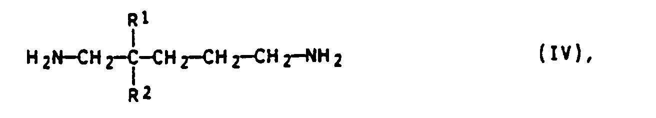 where R¹ and R² are identical or different and are each linear alkyl of 1 to 12 carbon atoms, branched alkyl of 3 to 12 carbon atoms, linear alkenyl of 2 to 12 carbon atoms or branched alkenyl of 4 to 12 carbon atoms and R¹ and R² together are alkylene of 4 to 7 carbon atoms which is unsubstituted or monosubstituted to pentasubstituted by C₁-C₄-alkyl, is reacted, in the presence or absence of a catalyst, with
a) urea and a primary and/or secondary alcohol R³OH and/or R⁴OH or with
b) urea and a primary and/or secondary alcohol R³OH and/or R⁴OH in the presence of an alkyl carbamate and/or dialkyl carbonate, where
R³ and R⁴ are identical or different and are each linear alkyl of 1 to 20 carbon atoms, branched alkyl of 3 to 20 carbon atoms or cycloalkyl of 5 to 12 carbon atoms,
and, if required, the ammonia formed is separated off.

6. A process for the preparation of a 2,2-dialkylpentane-1,5-diurethane of the formula (II) as claimed in claim 5, wherein a 2,2-dialkylpentylene-1,5-diamine (IV) is reacted with urea and an alcohol in a molar ratio of 1:1.5 to 10:2 to 50.

7. A process for the preparation of a 2,2-dialkylpentane-1,5-diurethane of the formula (II) as claimed in claim 5, wherein the alkyl carbamate corresponding to the alcohol is used in an amount of from 1 to 20 mol %, based on the 2,2-dialkylpentylene-1,5-diamine (IV).

8. Use of a 2,2-dialkylpentane-1,5-diurethane of the formula (II) for the preparation of a 2,2-dialkylpentane 1,5-diisocyanate of the formula (I).

9. Use of a 2,2-dialkylpentane diisocyanate as claimed in claim 1, with the exception of 2,2-dimethylpentane diisocyanate, for the production of adhesives, sealing compounds, coating materials and compact or cellular plastics by the polyisocyanate polyaddition method.

## Revendications

1. 2,2-dialkylpentane-1,5-diisocyanates de formule (I) 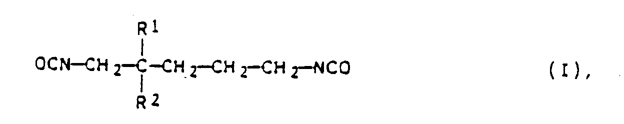 où R¹ et R² sont identiques ou différents et représentent:
un reste alkyle linéaire ayant 1 à 12 atomes de carbone,
un reste alkyle ramifié ayant 3 à 12 atomes de carbone,
un reste alcényle linéaire ayant 2 à 12 atomes de carbone
ou
un reste alcényle ramifié ayant 4 à 12 atomes de carbone,
à l'exclusion du 2,2-diméthylpentane-1,5-diisocyanate.

2. 2,2-dialkylpentane-1,5-diuréthannes de formule (II) 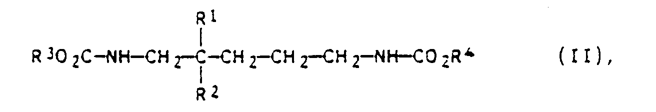 où R¹ et R² sont identiques ou différents et représentent:
un reste alkyle linéaire ayant 1 à 12 atomes de carbone,
un reste alkyle ramifié ayant 3 à 12 atomes de carbone,
un reste alcényle linéaire ayant 2 à 12 atomes de carbone,
un reste alcényle ramifié ayant 4 à 12 atomes de carbone,
les restes R¹ et R² représentent ensemble
un groupe alkylène ayant 4 à 7 atomes de carbone ou
un groupe alkylène ayant 4 à 7 atomes de carbone qui est substitué par 1 à 5 groupes alkyle en C₁ à C₄
et
R³ et R⁴ sont identiques ou différents et représentent:
un reste alkyle linéaire ayant 1 à 20 atomes de carbone,
un reste alkyle ramifié ayant 3 à 20 atomes de carbone ou
un reste cycloalkyle ayant 5 à 12 atomes de carbone.

3. Chlorures d'acide 2,2-dialkylpentane-1,5-dicarbamique de formule (III) 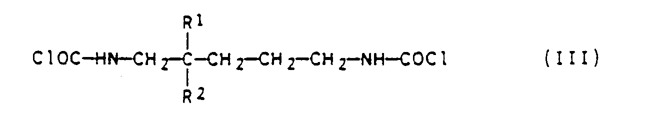 où R¹ et R² sont identiques ou différents et représentent:
un reste alkyle linéaire ayant 1 à 12 atomes de carbone,
un reste alkyle ramifié ayant 3 à 12 atomes de carbone,
un reste alcényle linéaire ayant 2 à 12 atomes de carbone ou
un reste alcényle ramifié ayant 4 à 12 atomes de carbone,
à l'exclusion du chlorure d'acide 2,2-diméthylpentane-1,5-dicarbamique.

4. Procédé pour la préparation de 2,2-dialkylpentane-1,5-diisocyanates de formule (I), caractérisé en ce qu'on coupe thermiquement des 2,2-dialkylpentane-1,5-diuréthannes de formule (II) en présence ou en l'absence de catalyseurs
a) en phase gazeuse à des températures supérieures à 300°C sous pression réduite ou
b) en phase liquide à des températures de 175 à 350°C.

5. Procédé pour la préparation de 2,2-dialkylpentane-1,5-diuréthannes de formule (II), caractérisé en ce qu'on fait réagir des 2,2-dialkylpentane-1,5-diamines de formule (IV) 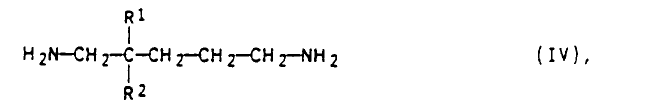 où R¹ et R² sont identiques ou différents et représentent:
un reste alkyle linéaire ayant 1 à 12 atomes de carbone,
un reste alkyle ramifié ayant 3 à 12 atomes de carbone,
un reste alcényle linéaire ayant 2 à 12 atomes de carbone,
un reste alcényle ramifié ayant 4 à 12 atomes de carbone,
les restes R¹ et R² représentent ensemble
un groupe alkylène ayant 4 à 7 atomes de carbone ou
un groupe alkylène ayant 4 à 7 atomes de carbone qui est substitué par 1 à 5 groupes alkyle en C₁ à C₄,
en présence ou en l'absence de catalyseurs avec
a) de l'urée et un alcool primaire et/ou secondaire R³OH et/ou R⁴OH ou avec
b) de l'urée et un alcool primaire et/ou secondaire R³OH et/ou R⁴OH, en présence d'esters alkyliques d'acide carbamique et/ou de dialkylcarbonates, tandis que
R³ et R⁴ sont identiques ou différents et représentent:
un reste alkyle linéaire ayant 1 à 20 atomes de carbone,
un reste alkyle ramifié ayant 3 à 20 atomes de carbone ou
un reste cycloalkyle ayant 5 à 12 atomes de carbone,
et on sépare éventuellement l'ammoniac formé.

6. Procédé pour la préparation de 2,2-dialkylpentane-1,5-diuréthannes de formule (II) selon la revendication 5, caractérisé en ce qu'on fait réagir les 2,2-dialkylpentane-1,5-diamines (IV) avec de l'urée et un alcool dans le rapport molaire de 1:1,5 à 10:2 à 50.

7. Procédé pour la préapration de 2,2-dialkylpentane-1,5-diuréthannes de formule (II) selon la revendication 5, caractérisé en ce qu'on emploie l'ester alkylique d'acide carbamique correspondant à l'alcool en une quantité de 1 à 20 % en moles, par rapport à la 2,2-dialkylpentane-1,5-diamine (IV).

8. Utilisation de 2,2-dialkylpentane-1,5-diuréthannes de formule (II) pour la préparation de 2,2-dialkylpentane-1,5-diisocyanates de formule (I).

9. Utilisation de 2,2-dialkylpentane-diisocyanates selon la revendication 1, à l'exception du 2,2-diméthylpentane-diisocyanate, pour la préparation d'adhésifs, de produits d'étanchéité, d'agents de revêtement et de matières plastiques compactes ou cellulaires par le procédé de polyaddition de polyisocyanate.
